# EUROPÄISCHE PATENTSCHRIFT

(11) **EP 1 314 447 B1**
(45) Veröffentlichungstag und Bekanntmachung des Hinweises auf die Patenterteilung: **02.02.2005**
(21) Anmeldenummer: 02090388.6
(22) Anmeldetag: 20.11.2002
(51) Int. Cl.: A61M 25/06

(54) **Einführkanüle zur Implantation von Kathetern**
Introducer sheath for introducing a catheter
Dispositif d'introduction pour introduire un cathéter

(30) Priorität: 20.11.2001 DE 10158289
(43) Veröffentlichungstag der Anmeldung: 28.05.2003
(73) Patentinhaber: BIOTRONIK Mess- und Therapiegeräte GmbH & Co Ingenieurbüro Berlin, 12359 Berlin (DE)
(72) Erfinder: Breskot, Tobias, 14612 Falkensee (DE); Schaldach, Max, Verstorben (DE)
(74) Vertreter: Eisenführ, Speiser & Partner

(56) Entgegenhaltungen:
- EP-A- 0 732 087
- WO-A-98/20812
- DE-A- 19 936 207
- US-A- 4 596 559
- US-A- 5 752 937
- US-A- 5 755 769

## Beschreibung

Die vorliegende Erfindung betrifft eine Einführkanüle zur Implantation von Kathetem mit einer Röhre mit einer Wandung, welche eine Innen- und eine Außenoberfläche aufweist und einen sowohl am proximalen sowie am distalen Ende offenen Hohlraum einschließt, und die dazu ausgestaltet ist, in einen Körper eingeführt zu werden, so dass mindestens ein Katheter zur lmplantation durch die Röhre einführbar ist. Die Röhre besitzt hierzu eine Länge besitzt, die derart bemessen ist, dass das proximale Ende der Röhre (2) beim Einführen eines Katheters außerhalb eines Körpers eines Patienten verbleibt.

Einführ-Kanülen werden dazu verwendet, Öffnungen in der Haut zum Einführen von verschiedenen medizinischen Vorrichtungen in weiches Gewebe oder Organe zu erzeugen. Beispielsweise kann eine Einführ-Kanüle durch einen Nadeleinstich eingeführt werden und ein Katheter kann dann mittels der Kanüle durch die Haut in den Patienten eingeführt werden. Nach dem Einführen des Katheters wird die Kanüle wieder aus dem Patienten entfernt. Im allgemeinen wird der Katheter dabei an ein medizinisches Gerät angeschlossen bevor die Kanüle entfernt wird. Um das Abziehen der Kanülen in proximaler Richtung trotz Hindernissen wie Elektrodensteckern oder Katheteranschlüssen zu ermöglichen, wird die Kanüle beim Abziehen gleichzeitig aufgerissen oder mittels eines proximal angebrachten Schneidwerkzeugs aufgeschnitten.

Aus US 4,377,165 ist eine auftrennbare Einführvorrichtung (ïlntroducerï) bekannt. Diese Vorrichtung weist längsseitig eine Sollbruchstelle auf, entlang der die Einführvorrichtung zu ihrer Entfernung aufgetrennt werden kann. Die Sollbruchstelle kann dabei beispielsweise als eine Kerbung, eine Perforation, als Löcher oder dergleichen ausgestaltet sein.

Dabei erweist es sich aber als nachteilig, dass die Stabilität der Einführvorrichtung verringert wird, um die Möglichkeit vorzusehen, die Einführvorrichtung aufzutrennen. Beispielsweise kann die Einführvorrichtung auf Grund ihrer verringerten Stabilität leicht und zum falschen Zeitpunkt aufgetrennt werden. Ein unerwünschtes Auftrennen der Einführvorrichtung kann beispielsweise auftreten, wenn die Vorrichtung zunächst in den Patienten eingeführt wird, bevor der Katheter komplett durch die Einführvorrichtung in den Patienten eingeführt wurde, bevor der Katheter an die gewünschte medizinische Vorrichtung angeschlossen wurde oder bevor die Einführvorrichtung aus dem Patienten und vom Katheter entfernt wurde.

Ferner bietet die oben beschriebene auftrennbare Einführvorrichtung kein zuverlässiges Auftrennen und tendiert dazu, scharfe Kanten aufzuweisen, wenn sie aufgetrennt wird.

US 5,752,937 zeigt eine medizinische Einführvorrichtung. Diese Einführvorrichtung weist dabei eine Röhre mit einem Verstärkungsstreifen oder einem Draht auf, welche in das Material der Röhre eingelassen sind und sich im wesenttichen entlang der Längsachse der Röhre erstrecken. Der Verstärkungsstreifen oder der Draht kann dabei aus jedem geeigneten Material hergestellt werden, das als Schneidemittel verwendet werden kann, welches entlang der Längsachse der Röhre gezogen werden kann, um die Einführvorrichtung aufzuschneiden und dadurch die Einführvorrichtung zum Entfernen von dem Katheter aufzutrennen. Der Verstärkungsstreifen oder der Draht weist dabei eine größere Scherkraft als das Material der Röhre der Einführvorrichtung auf. Das Aufschneiden der Einführvorrichtung durch den Verstärkungsstreifen oder den Draht wird durch die Differenz der Scherstärken erreicht.

Hierbei erweist es sich jedoch als nachteilig, dass die gesamte Längswandung der Röhre der Einführvorrichtung gleichzeitig durchschnitten werden muss.

Aus der WO 98/20812 ist ein Katheter zum Einführen eines Stents bekannt, bei dem lediglich das distale Ende der Katheterröhre mit Hilfe eines Drahtes aufzuschlitzen ist, der über den größten Teil. der Katheterlänge im Inneren des Katheters geführt ist und im Bereich des distalen Endes durch ein Loch auf die Katheteraußenseite tritt. Auf der Katheteraußenseite ist der Draht völlig frei und ungeführt.

Aufgabe der Erfindung ist es somit, eine Vorrichtung zum Implantieren von Kathetern vorzusehen, welche sich leicht beim Entfernen aus dem Körper auftrennen lässt, ohne dabei die nötigen Stabilitätserfordernisse zu vernachlässigen.

Diese Aufgabe wird durch eine Einführkanüle zum Implantieren von Kathetern der eingangs genannten Art mit den kennzeichnenden Merkmalen des beigefügten Anspruchs 1 gelöst.

Der Erfindung liegt dabei der Gedanke zu Grunde, eine Vorrichtung zur Implantation von Kathetern vorzusehen, welche eine Röhre mit einer Wandung sowie einen Draht aufweist. Die Röhre ist dabei dazu vorgesehen in einen Körper eingeführt zu werden, so dass mindestens ein Katheter zur Implantation durch die Röhre eingeführt werden kann. Die Wandung der Röhre weist eine Innen- und Außenoberfläche auf. Der Draht

ist an seinem ersten Ende im Bereich des proximalen Endes der Röhre an der Innen- oder Außenoberfläche der Wandung befestigt und/oder in die Wandung eingelassen, der Draht wird dann in der Nähe der Außenoberfläche entlang der Längsrichtung der Röhre vom proximalen zum distalen Ende der Röhre geführt und anschließend in der Nähe der jeweils anderen Oberfläche der Wandung entlang der Längsrichtung vom distalen zum proximalen Ende der Röhre zurückgeführt.

Die mit der Erfindung einhergehenden Vorteile liegen insbesondere darin, dass die Implantationsvorrichtung durch Ziehen des Drahts vom distalen zum proximalen Ende hin aufgeschnitten wird und somit zunächst der gesamten Länge nach aufgetrennt wird. Dadurch wird die Reibung zwischen der Implantationsvorrichtung und dem implantierten Katheter verringert und das Abziehen der Implantationsvorrichtung nach erfolgter Implantation des Katheters führt nicht zu einer Gefährdung der Implantationslage und/oder der Implantationsposition des Katheters.

Durch das nach und nach erfolgende Aufschneiden der Röhre der Länge nach werden der Draht und die Wandung der Röhre wesentlich geringeren Spannungen ausgesetzt, wodurch eine elastische Verformung verhindert wird, die wiederum die Implantationsposition des implantierten Katheters gefährden kann.

Die Stabilität der Implantationsvorrichtung wird ferner dadurch gewährleistet, dass die Röhre einstückig gefertigt werden kann.

Das zweite Ende des Drahtes ist am proximalen Ende der Röhre frei zugänglich. Dadurch wird erreicht, dass die Bedienung beim Aufschneiden der Vorrichtung vereinfacht wird.

Bei einer bevorzugten Ausgestaltung der Erfindung weist die Vorrichtung zwei Stege am proximalen Ende der Röhre auf. Diese Stege dienen dazu, die Handhabbarkeit der Vorrichtung insbesondere beim Aufschneiden der Vorrichtung zu vereinfachen. Das Vorsehen der beiden Stege führt dazu, dass der Bediener die Vorrichtung mit Hilfe der beiden Stege beim Aufschneiden besser festhalten kann.

Bei einer weiteren bevorzugten Ausgestaltung der Erfindung weist der Draht an seinem zweiten Ende einen Griff auf. Durch Ziehen an dem Griff kann die lmplantationsvorrichtung vom distalen zum proximalen Ende aufgeschnitten werden, was eine Erleichterung der Handhabung mit sich bringt.

Die Außenoberfläche der Wandung der Röhre weist einen Längsschlitz auf, der dazu ausgestaltet ist, den Draht zu führen. Durch das Führen des Drahtes in dem Längsschlitz entlang der Außenoberfläche der Wandung und das Rückführen des Drahtes an der Innenoberfläche der Wandung wird vermieden, dass der Draht die Implantationsvorrichtung verlässt und in die umliegenden Gefäßwände einschneiden kann.

Die Länge der Röhre bemisst sich nach deren Zweck, nämlich als Einführkatheter zu dienen. Geeignete Längen liegen im Bereich zwischen 400 und 500 Millimeter.

Im Nachfolgenden wird ein Ausführungsbeispiel anhand der Figur näher erläutert.

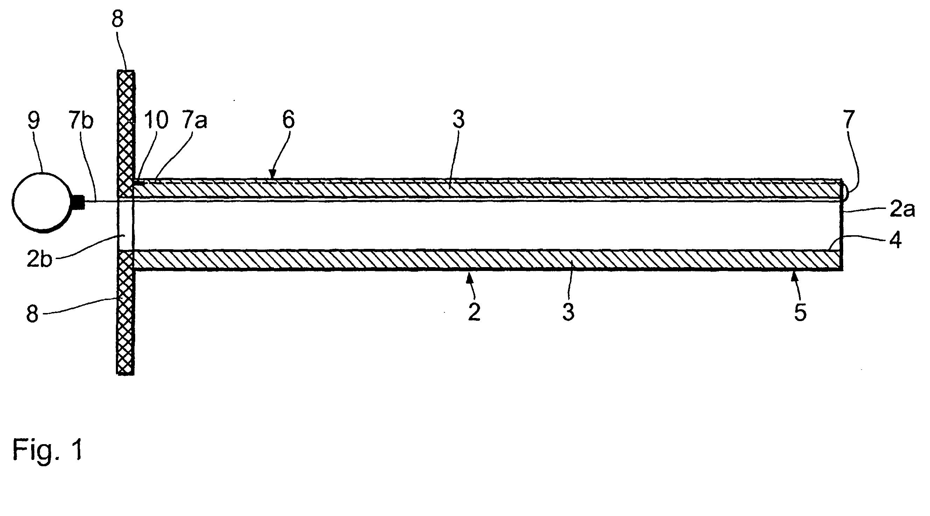

Fig. 1 zeigt eine Schnittansicht einer Implantafionsvorrichtung.

In Fig. 1 ist eine Röhre 2 mit einer Wandung 3 sowie zwei an ihrem proximalen Ende 2b angeordneten Stegen 8 gezeigt. Die Röhre 2 weist ferner an einer Außenoberfläche 5 der Wandung 3 einen Längsschlitz 6 entlang ihrer Längsrichtung auf. Das erste Ende 7a eines Drahtes 7 wird an einer Stelle 10 am proximalen Ende 2b der Röhre 2 befestigt. Der Draht 7 wird dann in dem Längsschlitz 6 von dem proximalen Ende 2b zu dem distalen Ende 2a der Röhre 2 geführt. Anschließend wird der Draht 7 am distalen Ende 2a der Röhre 2 um das distale Ende 2a herumgeführt und an der Innenoberfläche 4 der Wandung 3 vom distalen Ende 2a zum proximalen Ende 2b der Röhre 2 zurückgeführt.

Die Länge des Drahtes 7 ist dabei derart gewählt, dass das zweite Ende 7b des Drahtes 7 aus dem proximalen Ende 2b des Rohres 2 herausragt. An diesem zweiten Ende 7b des Drahtes 7 wird vorzugsweise ein Griff 9 befestigt.

Durch Ausüben eines Zugs auf das Seil wird der Draht 7 in proximaler Richtung gezogen und durchschneidet dabei die Wandung 3 der Röhre 2 der Länge nach. Vorzugsweise wird die Wandung 3 der Röhre 2 der Implantationsvorrichtung vor dem Abziehen der Implantationsvorrichtung der Länge nach aufgetrennt. Dadurch wird die Reibung zwischen der Implantationsvorrichtung und einem zu implantierenden Katheter verringert, so dass das Abziehen der Implantationsvorrichtung die Implantationsposition des Katheters nicht beeinträchtigt. Durch das nach und nach erfolgte Aufschneiden der Röhre 2 der Länge nach werden der Draht 7 und die Wandung 3 der Röhre 2 wesentlich geringeren Spannungen ausgesetzt, wodurch eine elastische Verformung verhindert wird, die wiederum die Implantationsposition des implantierten Katheters gefährden könnte

Durch das Führen des Drahtes 7 in dem Längsschlitz 6 entlang der Außenoberfläche 5 der Wandung 3 und das Rückführen des Drahtes 7 an der Innenoberfläche 4 der Wandung 3 wird vermieden, dass der Draht 7 die Implantationsvorrichtung verlässt und in die umliegenden Gefäßwände einschneidet.

## Patentansprüche

1. Einführkanüle zur Implantation von Kathetern,
mit einer Röhre (2) mit einem offenen proximalen und einem offenen distalen Ende sowie einer Wandung (3), welche eine Innen- und eine Außenoberfläche (4, 5) aufweist und einen sowohl am proximalen sowie am distalen Ende offenen Hohlraum zum Hindurchführen eines Katheters einschließt, wobei die Röhre (2) dazu ausgestaltet ist, in einen Körper eingeführt zu werden, so dass ein Katheter zur Implantation durch die Röhre (2) einführbar ist, und wobei die Röhre eine Länge besitzt, die derart bemessen ist, dass das proximale Ende der Röhre (2) beim Einführen eines Katheters außerhalb eines Körpers eines Patienten verbleibt, und **gekennzeichnet durch** einen Draht (7), welcher ein erstes Ende (7a) aufweist, das zumindest im Bereich des proximalen Endes (2b) der Röhre (2) in der Nähe der Innen- oder der Außenoberfläche (4,5) der Wandung (3) befestigt ist,
wobei sich der Draht vom seinem befestigten ersten Ende (7a) am proximalen Ende (2b) der Röhre (2) ausgehend in der Nähe der Außenoberfläche (5) der Wandung (3) entlang der Längsrichtung der Röhre (2) zu deren distalem Ende (2b, 2a) erstreckt und von dort in der Nähe Innenoberfläche (4) in dem Hohlraum der Röhre entlang der Längsrichtung vom distalen zum proximalen Ende (2a, 2b) der Röhre (2) bis zu einem zweiten, frei zugänglichen Ende (7b) des Drahts (7) am proximalen Ende (2b) der Röhre (2) zurückgeführt ist, wobei
entweder der Draht (7) auf seiner Länge vom proximalen Ende der Röhre (3) zu deren distalem Ende zumindest teilweise in die Wandung (3) eingebettet ist,
oder in der Außenoberfläche (5) der Wandung (3) ein Längsschlitz (6) eingelassen ist, der dazu ausgestaltet ist, den Draht zu führen.

2. Einfürhkanüle nach Anspruch 1, **dadurch gekennzeichnet, dass** die Länge der Röhre (2) zwischen 400 und 500 mm beträgt.

3. Einführkanüle nach Anspruch 1, **gekennzeichnet durch** zwei Stege (8) am proximalen Ende (2b) der Röhre (2).

4. Einführskanüle nach Anspruch 1, **dadurch gekennzeichnet, dass** der Draht (7) an seinem zweiten Ende (7b) einen Griff (9) aufweist

## Claims

1. Insertion cannula for the implantation of catheters having a tube (2) with an open proximal end and an open distal end and a wall (3) which has an inside surface and an outside surface (4, 5) and encloses a cavity open both at the proximal end and at the distal end for feeding through a catheter, wherein the tube (2) is adapted to be inserted into a body so that a catheter can be introduced for the implantation procedure through the tube (2), and wherein the tube is of a length which is such that the proximal end of the tube (2) remains outside a body of a patient on insertion of a catheter, **characterised by** a wire (7) which has a first end (7a) fastened, at least in the region of the proximal end (2b) of the tube (2), in the proximity of the inside or outside surface (4, 5) of the wall (3), the wire, starting from its fastened first end (7a) at the proximal end (2b) of the tube (2), extends in the proximity of the outside surface (5) of the wall (3) along the longitudinal direction of the tube (2) to the distal end (2b, 2a) thereof and from there is guided back in the proximity of the inside surface (4) in the cavity of the tube along the longitudinal direction from the distal end to the proximal end (2a, 2b) of the tube (2) up to a second, freely accessible end (7b) of the wire (7) at the proximal end of the tube (2), wherein either the wire (7), over its length from the proximal end of the tube (2) to the distal end thereof, is at least partially embedded in the wall (3) or let into the outside surface (5) of the wall (3) is a longitudinal slot (6) which is adapted to guide the wire (7).

2. Insertion cannula according to claim 1, **characterised in that** the length of the tube (2) is between 400 and 500 mm.

3. Insertion cannula according to claim 1, **characterised by** two arms (8) at the proximal end (2b) of the tube (2).

4. Insertion cannula according to claim 1, **characterised in that** at its second end (7b) the wire (7) has a handle (9).

## Revendications

1. Canule d'introduction pour implanter des cathéters,
comportant un tube (2) avec une extrémité proximale ouverte et une extrémité distale ouverte, ainsi qu'une paroi (3) qui présente des surfaces intérieure et extérieure (4, 5) et qui enferme une cavité ouverte aussi bien à l'extrémité proximale qu'à l'extrémité distale et destinée à faire traverser un cathéter, ledit tube (2) étant réalisé pour être introduit dans un corps, de sorte qu'un cathéter à implanter peut être introduit à travers le tube (2), et le tube possédant une longueur qui est dimensionnée de telle sorte que l'extrémité proximale du tube (2) reste à l'extérieur du corps d'un patient lors de l'introduction d'un cathéter, **caractérisée par** un fil (7) qui présente une première extrémité (7a) qui est fixée à proximité de la surface intérieure ou extérieure (4, 5) de la paroi (3) au moins dans la zone de l'extrémité proximale (2b) du tube (2), le fil s'étendant à partir de sa première extrémité fixée (7a), à l'extrémité proximale (2b) du tube (2), à proximité de la surface extérieure (5) de la paroi (3) le long de la direction longitudinale du tube (2) jusqu'à son extrémité distale (2b, 2a) et retournant depuis ici à proximité de la surface intérieure (4) dans la cavité du tube le long de la direction longitudinale depuis l'extrémité distale vers l'extrémité proximale (2a, 2b) du tube (2) jusqu'à une deuxième extrémité (7b), librement accessible, du fil (7) à l'extrémité proximale (2b) du tube (2), et
soit le fil (7) est noyé au moins partiellement dans la paroi sur sa longueur depuis l'extrémité proximale du tube (2) jusqu'à son extrémité distale,
soit une fente longitudinale (6) est ménagée dans la surface extérieure (5) de la paroi (3), fente qui est conçue pour guider le fil.

2. Canule d'introduction selon la revendication 1, **caractérisée en ce que** la longueur du tube (2) est comprise entre 400 et 500 mm.

3. Canule d'introduction selon la revendication 1, **caractérisée par** deux barrettes (8) à l'extrémité proximale (2b) du tube (2).

4. Canule d'introduction selon la revendication 1, **caractérisée en ce que** le fil (7) comprend une manette (9) à sa deuxième extrémité (7b).
